(19) Europäisches Patentamt
European Patent Office
Office européen des brevets

(11) Veröffentlichungsnummer : **0 124 002**
**B1**

(12) # EUROPÄISCHE PATENTSCHRIFT

(45) Veröffentlichungstag der Patentschrift : **12.11.86**

(21) Anmeldenummer : **84104277.3**

(22) Anmeldetag : **16.04.84**

(51) Int. Cl.⁴ : **C 07 B 39/00**// C07C43/225, C07C65/21, C07C149/34, C07D317/46, C07D319/20

(54) **Verfahren zur Herstellung von aromatischen Verbindungen, die über ein Heteroatom gebundene perfluorierte Seitenketten enthalten.**

(30) Priorität : **27.04.83 DE 3315147**

(43) Veröffentlichungstag der Anmeldung : **07.11.84 Patentblatt 84/45**

(45) Bekanntmachung des Hinweises auf die Patenterteilung : **12.11.86 Patentblatt 86/46**

(84) Benannte Vertragsstaaten : **CH DE FR GB IT LI**

(56) Entgegenhaltungen :
**FR-A- 2 329 625**
**THE JOURNAL OF ORGANIC CHEMISTRY, Band 44, Nr. 16, 3. August 1979, A.E. Feiring, Chemistry in Hydrogen Fluoride. 7. A Novel Synthesis of Aryl Trifluoromethyl Ethers, Seiten 2907-2910.**

(73) Patentinhaber : **BAYER AG**
**Konzernverwaltung RP Patentabteilung**
**D-5090 Leverkusen 1 Bayerwerk (DE)**

(72) Erfinder : **Marhold, Albrecht, Dr.**
**Carl-Duisberg-Strasse 329**
**D-5090 Leverkusen 1 (DE)**
Erfinder : **Klauke, Erich, Dr.**
**Eichendorffweg 8**
**D-5068 Odenthal (DE)**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 124 002 B1

Jouve, 18, rue St-Denis, 75001 Paris, France

**Beschreibung**

Die vorliegende Erfindung betrifft ein Verfahren zur Herstellung von aromatischen Verbindungen, die über ein Heteroatom gebundene perfluorierte Seitenketten enthalten aus aromatischen Verbindungen, die über ein Heteroatom gebundene perhalogenierte, aber nur partiell fluorierte Seitenketten enthalten, durch Behandlung mit einem Katalysator.

Aromatische Verbindungen, die über ein Heteroatom gebundene perfluorierte Seitenketten enthalten, sind wichtige Zwischenprodukte zur Herstellung von Herbiz — den (s. DE-OS 28 48 531) und Insektiziden (s. DE-OS 28 37 524 und DE-OS 30 23 328).

Es ist bekannt, daß man aromatische Verbindungen, die über ein Heteroatom gebundene perfluorierte Seitenketten enthalten, herstellen kann, indem man die entsprechenden perchlorierten Verbindungen mit Fluorwasserstoff umsetzt. Nachteilig hierbei ist, daß im allgemeinen ein Überschuß an Fluorwasserstoff eingesetzt werden muß, der nur schwierig zurückzugewinnen ist oder vernichtet werden muß. Weiterhin nachteilig ist, daß sonstige vorhandene fluorierbare Gruppen, z. B. Säurechloridgruppen, ebenfalls fluoriert werden, was im allgemeinen unerwünscht ist und einen zusätzlichen Verbrauch an Fluorwasserstoff bedeutet. So ist beispielsweise aus der DE-OS 21 17 650 bekannt, daß man bei der Fluorierung von 4-Trichlormethoxybenzoylchlorid mit Fluorwasserstoff je nach Reaktionsführung 4-Trifluormethoxybenzoylfluorid und/oder 4-Difluorchlormethoxybenzoylfluorid enthalten kann, nicht aber 4-Trifluormethoxybenzoylchlorid.

Weiterhin ist aus der FR-A-2 329 625 bekannt, daß man aromatische Verbindungen, die direkt an den aromatischen Kern gebundene perhalogenierte, aber nur partiell fluorierte Seitenketten enthalten, mit Hilfe von bestimmten Lewis-Säuren in entsprechende perfluorierte Seitenketten enthaltende Verbindungen überführen kann. Die Anwendung einer solchen Reaktion auf aromatische Ausgangsverbindungen, die über Sauerstoff-, Schwefel- und/oder Stickstoffatome gebundene perhalogenierte, aber nur partiell fluorierte Seitenketten enthalten zur Herstellung von aromatischen Verbindungen, die über solche Heteroatome gebundene perfluorierte Seitenketten enthalten, hat aus mehreren Gründen nicht nahegelegen. Beispielsweise ist in diesem Fall mit der Bildung einer Vielzahl unterschiedlicher Verbindungen zu rechnen. Außerdem ist gemäß J. Org. Chem., Vol. 44, No. 16, 1979, Seiten 2907 bis 2910 mit Nebenreaktionen in erheblichem Umfang (insbesondere mit Teerbildung und Spaltungsreaktionen) zu rechnen, wenn man z. B. Difluorchlormethoxygruppen enthaltende Aromaten mit Halogenatomen enthaltenden Reagentien und/oder Katalysatoren behandelt.

Es wurde nun ein Verfahren zur Herstellung von aromatischen Verbindungen, die über ein Heteroatom gebundene perfluorierte Seitenketten enthalten, gefunden, das dadurch gekennzeichnet ist, daß man aromatische Verbindungen, die über Sauerstoff-, Schwefel- und/oder Stickstoffatome gebundene perhalogenierte, aber nur partiell fluorierte Seitenketten enthalten, mit einem Lewis-Säure-Katalysator behandelt.

In das erfindungsgemäße Verfahren können die verschiedensten aromatischen Verbindungen eingesetzt werden, die über Sauerstoff-, Schwefel- und/oder Stickstoffatome gebundene perhalogenierte, aber nur partiell fluorierte Seitenketten enthalten. Diese aromatischen Verbindungen können weitere Substituenten enthalten, beispielsweise Alkyl-, Aryl-, Carbonsäurefluorid-, Carbonsäurechlorid-, Nitro-, Sulfochlorid-, Cyanid-, O-Alkyl-, O-Aryl- und/oder Halogen-Gruppen. Es kann dabei ein derartiger Substituent vorhanden sein, es können aber auch unabhängig voneinander mehrere, beispielsweise 2 bis 4 gleichartige oder verschiedenartige dieser Substituenten vorhanden sein. Die aromatischen Verbindungen können auch mit einem carbocyclischen Ring substituiert sein.

Von den perhalogenierten, aber nur partiell fluorierten Seitenketten können eine oder mehrere pro Molekül aromatischer Verbindungen enthalten sein. Diese Seitenketten können auch als Zyklus vorliegen. Dieser ist dann vorzugsweise über zwei Heteroatome an den aromatischen Kern gebunden. Die perhalogenierten, aber nur partiell fluorierten Seitenketten enthalten vorzugsweise 1 oder 2 C-Atome und als Halogen neben Fluor vorzugsweise Chlor und/oder Brom.

Eine bevorzugte Gruppe von in das erfindungsgemäße Verfahren einsetzbaren Verbindungen sind solche der Formel

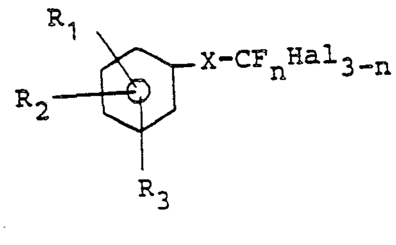

(I)

in der unabhängig voneinander

$R_1$ für Wasserstoff, $C_1$-$C_4$-Alkyl, $C_6$-$C_8$-Aryl, COF, COCl, $NO_2$, $SO_2Cl$, CN, O-$C_1$-$C_4$-alkyl, O-$C_6$-$C_8$-Aryl, F, Cl oder Br steht,

$R_2$ für Wasserstoff, $NO_2$, $SO_2Cl$, F, Cl oder Br steht,
$R_3$ für Wasserstoff, COF, COCl, F, Cl oder Br steht, oder
$R_1$ und $R_2$ gemeinsam für einen carbocyclischen Ring mit 3 bis 4 C-Atomen stehen,
X für O, S oder N-$C_1$-$C_4$-Alkyl steht,
n für 1 oder 2 steht und
Hal für Cl und/oder Br steht.

Eine weitere bevorzugte Gruppe von in das erfindungsgemäße Verfahren einsetzbaren Verbindungen sind solche der Formel

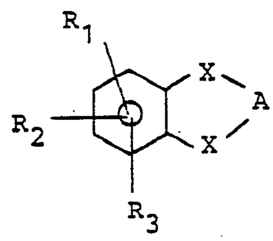

(II)

in der
$R_1$, $R_2$, $R_3$ und X die bei Formel (I) angegebene Bedeutung haben und
A für CFHal, $C_2FHal_3$, $C_2F_2Hal_2$ oder $C_2F_3Hal$ steht, wobei Hal für Cl und/oder Br steht.

Besonders bevorzugt werden in das erfindungsgemäße Verfahren 2-Fluor-2-chlor-benzodioxol, 3-Difluorchlormethoxy-benzoylfluorid, 2-Difluormethoxy-trichlormethoxybenzol, 6-Nitro-2-chlor-2,3,3-trifluor-benzo-1,4-dioxen, 4-Difluorchlormethoxy-nitrobenzol, 3-Difluorchlormethoxy-nitrobenzol, 4-Difluorchlormethoxy-3-methyl-nitrobenzol, 4-Difluorchlormethoxy-phenylchlorkohlensäureester, 3-Difluorchlormethoxy-2,4-dichlortoluol, 3-Difluorchlormethoxy-4,6-dichlortoluol, 3-Difluorchlormethoxy-2,4,6-trichlortoluol und 2-Chlor-2,3,3-trifluor-1,4-benzodioxen eingesetzt.

Die in das erfindungsgemäße Verfahren einzusetzenden aromatischen Verbindungen, die über Sauerstoff-, Schwefel- und/oder Stickstoffatome gebundene perhalogenierte, aber nur partiell fluorierte, Seitenketten enthalten, können beispielsweise auf einfache Weise durch Fluorierung der entsprechenden perhalogenierten, aber fluorfreien Verbindungen erhalten werden. Diese Fluorierung kann z. B. mit Fluorwasserstoff oder Alkalifluoriden durchgeführt werden. Da Fluorwasserstoff im allgemeinen befähigt ist, sämtliche vorhandenen Halogenatome gegen Fluoratome auszutauschen, sind bei der Fluorierung mit Fluorwasserstoff besondere Maßnahmen zu ergreifen, um Verbindungen mit nur partiell fluorierter Seitenkette zu erhalten. Alkalifluoride sind im allgemeinen nur befähigt, einen Teil der vorhandenen Halogenatome gegen Fluoratome auszutauschen. Aus diesem Grund wird die Fluorierung von aromatischen Verbindungen, die über Sauerstoff-, Schwefel- und/oder Stickstoffatome gebundene perhalogenierte, aber fluorfreie Seitenketten enthalten, bevorzugt mit Alkalifluoriden durchgeführt.

Als Katalysatoren kommen für das erfindungsgemäße Verfahren insbesondere die Halogenide der Elemente Bor, Aluminium, Zinn, Arsen, Antimon, Titan, Molybdän und Eisen in Frage. Bevorzugt sind die Fluoride, Chloride und Bromide dieser Elemente. Besonders bevorzugt sind Aluminiumtrichlorid, Aluminiumtribromid, Bortrichlorid, Bortrifluorid, Arsenpentachlorid, Antimonpentachlorid, Molybdänpentachlorid, Titantetrachlorid und Eisentrichlorid. Sofern Aluminiumchlorid als Katalysator eingesetzt wird, wird dieses vorzugsweise im Gemisch mit Natriumchlorid verwendet.

Die Katalysatoren können beispielsweise in Mengen von 0,001 bis 0,1 Mol, bezogen auf 1 Mol aromatische Verbindung eingesetzt werden. Bevorzugt ist der Einsatz von 0,005 bis 0,05 Mol Katalysator, bezogen auf aromatische Verbindung.

Die Behandlung mit dem Katalysator kann beispielsweise bei Temperaturen im Bereich von 0 bis 150 °C durchgeführt werden. Bevorzugt sind Temperaturen im Bereich von 20 bis 140 °C.

Die Reaktionszeit kann beispielsweise im Bereich von 5 bis 600 Minuten liegen. Im allgemeinen sind Reaktionszeiten zwischen 30 und 200 Minuten ausreichend.

Das erfindungsgemäße Verfahren wird im allgemeinen ohne Lösungs- und Verdünnungsmittel durchgeführt. Es ist aber auch möglich, in Gegenwart von gegenüber den Katalysatoren inerten Lösungsmitteln zu arbeiten. Geeignete Lösungsmittel sind beispielsweise Chlorbenzol und Dichlorbenzol.

Da die Katalysatoren in der Regel wasserempfindlich sind ist es vorteilhaft, das erfindungsgemäße Verfahren unter weitgehendem oder völligem Ausschluß von Feuchtigkeit durchzuführen.

Das erfindungsgemäße Verfahren wird im allgemeinen bei Normaldruck durchgeführt, es kann jedoch auch bei erniedrigtem oder erhöhtem Druck durchgeführt werden. Erniedrigte Drucke sind z. B. dann von Vorteil, wenn man relativ hochsiedende aromatische Verbindungen mit über ein Heteroatom gebundenen perfluorierten Seitenketten herstellt und diese während der Reaktion kontinuierlich durch Destillation entfernen möchte. Erhöhte Drucke sind z. B. dann von Vorteil, wenn der Siedepunkt einer Komponente des Reaktionsgemisches bei Normaldruck niedriger liegt als die gewünschte Reaktionstemperatur.

Das erfindungsgemäße Verfahren läßt sich an zwei ausgewählten Beispielen durch folgende

Reaktionsgleichungen erläutern :

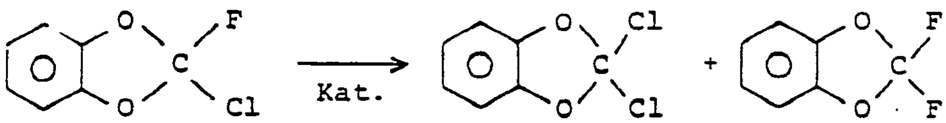


(1)

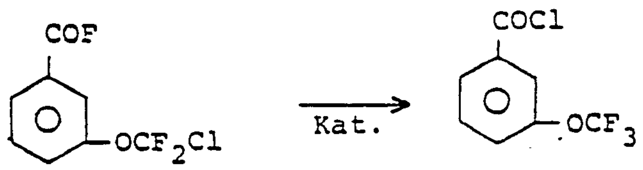


(2) .

Nach der Durchfürung des erfindungsgemäßen Verfahrens liegt im allgemeinen ein Gemisch vor, das als Komponente A die hergestellte, über Sauerstoff-, Schwefel- und/oder Stickstoffatome gebundene, perfluorierte Seitenketten enthaltende aromatische Verbindung, als Komponente(n) B die entsprechende(n) perhalogenierte, fluor- freie Seitenketten enthaltende aromatische Verbindung und/oder gegenüber der Ausgangsverbindung fluorärmere aromatische Verbindung(en) und gegebenenfalls als Komponente(n) C Lösungsmittel, unumgesetzte Ausgangsverbindung und/oder Nebenprodukte enthält.

Die Aufarbeitung dieser Gemische kann beispielsweise durch Destillation erfolgen. Die Komponente A hat dabei im allgemeinen den niedrigsten Siedepunkt, so daß diese während oder nach der Durchführung des erfindungsgemäßen Verfahrens als erste abgetrennt werden kann. Sofern ein unterhalb der Komponente A siedendes Lösungsmittel eingesetzt wurde, ist dieses vor der destillativen Abtrennung der Komponente A zu entfernen. Im allgemeinen ist es vorteilhaft, aus dem nach der Abtrennung der Komponente A vorliegenden Rückstand auch die Komponente(n) B und, soweit vorhanden, unumgesetzte Ausgangsverbindungen, durch Destillation abzutrennen. Die abgetrennte(n) Komponente(n) B können nach Fluorierung, beispielsweise mittels Alkalifluoriden, einem weiteren Ansatz zur Durchführung des erfindungsgemäßen Verfahrens zugefügt werden. Die abgetrennten unumgesetzten Ausgangsverbindungen können direkt einem weiteren Ansatz zur Durchführung des erfindungsgemäßen Verfahrens zugefügt werden.

Wenn das Einsatzmaterial für das erfindungsgemäße Verfahren weitere Fluoratome enthält, als die in den partiell fluorierten Seitenketten vorliegenden Fluoratome, z. B. beim Einsatz von 3-Difluorchlormethoxy-benzoylfluorid, fallen häufig Reaktionsgemische an, die keine Komponente B enthalten, sondern neben dem gewünschten Endprodukt (Komponente A), beim Einsatz von 3-Difluorchlormethoxy-benzoylfluorid ist das 3-Trifluormethoxy-benzoylchlorid, allenfalls unumgesetztes Ausgangsprodukt und gegebenenfalls geringe Mengen an Nebenprodukten. In solchen Fällen ist die Aufarbeitung besonders einfach, da nach der destillativen Abtrennung der Komponente A der Rückstand verworfen oder einem weiteren Ansatz zur Durchführung des erfindungsgemäßen Verfahrens zugefügt werden kann.

In vielen Fällen ist es vorteilhaft, die Komponente A während der Reaktion kontinuierlich durch Destillation aus dem Reaktionsgemisch zu entfernen.

Die eingesetzten Katalysatoren sind im allgemeinen schwerer flüchtig als die Komponenten A und B. Sie verbleiben deshalb bei einer destillativen Aufarbeitung im allgemeinen im Rückstand und können mit diesem entweder erneut einem weiteren Ansatz zur Durchführung des erfindungsgemäßen Verfahrens zugefügt oder verworfen werden. In Fällen, in denen der eingesetzte Katalysator durch seine Flüchtigkeit eine destillative Aufarbeitung stören würde, empfiehlt es sich, den Katalysator vor der Destillation durch Zugabe von wenig Wasser zu zersetzen.

Es ist ausgesprochen überraschend, daß es mit dem erfindungsgemäßen Verfahren gelingt, aromatische Verbindungen mit über Sauerstoff-, Schwefel- und/oder Stickstoffatome gebundenen perfluorierten Seitenketten in guten Ausbeuten zu erhalten, denn bei der Herstellung dieser Verbindungen durch Fluorierung mit Fluorwasserstoff sind die Ausbeuten niedrig und es fallen größere Mengen an verharzten Nebenprodukten an, die nicht mehr verwertbar sind. Beim erfindungsgemäßen Verfahren werden dagegen nur wenig oder gar keine verharzten Produkte gebildet und unumgesetztes Ausgangsprodukt und/oder niedriger oder nicht fluorierte Nebenprodukte, letztere nach einer Nachfluorierung, können einem weiteren Ansatz zur Durchführung des erfindungsgemäßen Verfahrens zugefügt werden. Siehe hierzu beispielsweise die Beispiele 8 und 9.

Die folgenden Beispiele erläutern das erfindungsgemäße Verfahren, ohne es in irgendeiner Weise zu beschränken.

## Beispiele

### Beispiel 1

(Herstellung des Ausgangsproduktes 2-Fluor-2-chlorbenzodioxol, nicht erfindungsgemäß)

In einer getrockneten Rührapparatur, die mit Rückflußkühler und Trockenrohr versehen war, wurden 40 g Kaliumfluorid, das zuvor bei 400 °C getrocknet wurde, und 150 ml getrocknetes Acetonitril vorgelegt. Zu diesem Gemisch wurden bei Raumtemperatur 100 g (0,52 Mol) 2,2-Dichlorbenzodioxol zugetropft und anschließend das Gemisch für 2 Stunden zum Sieden am Rückfluß erhitzt. Anschließend wurd abgekühlt, die festen Bestandteile des Reaktionsgemisches abgesaugt und das Filtrat fraktioniert destilliert. Es wurden 76 g 2-Fluor-2-chlor-benzodioxol (= 91 % der Theorie) mit einem Siedepunkt von 67 bis 70 °C bei 20 mbar und mit einem Brechungsindex von $n_D^{20}$ von 1,4920 erhalten.

## Beispiel 2

In einer Rührapparatur, die mit einer Kolonne und Rückflußteiler versehen war, wurden 87 g (0,5 Mol) des gemäß Beispiel 1 hergestellten 2-Fluor-2-chlor-benzodioxols vorgelegt, 1 ml Antimonpentachlorid zugegeben und für 5 Stunden auf 100 °C erhitzt. Anschließend wurde die Sumpftemperatur erhöht und das entstandene 2,2-Difluorbenzodioxol über die Kolonne abdestilliert. Es wurden 38 g 2,2-Difluorbenzodioxol mit einem Siedepunkt von 130 bis 131 °C bei 1 030 mbar und mit einem Brechungsindex von $n_D^{20}$ von 1,4430 erhalten. Der Sumpf bestand überwiegend aus 2,2-Dichlorbenzodioxol, das ebenfalls durch Destillation gewonnen wurde. Eine gaschromatographische Analyse der Reaktionsmischung vor der Destillation zeigte an, daß dort praktisch nur die beiden Reaktionsprodukte vorlagen und kein Ausgangsmaterial mehr vorhanden war.

## Beispiel 3

In einer Rührapparatur wurden 50 g 3-Difluorchlor-methoxy-benzoylfluorid vorgelegt und 0,5 ml Antimonpentachlorid zugegeben. Anschließend wurde die Mischung für 3 Stunden bei 100 °C und unter Feuchtigkeitsausschluß gerührt, abgekühlt und der Umwandlungsgrad durch $F^{19}$-NMR-Spektroskopie bestimmt. Es lag demnach ein Gemisch von 88 Gew.-% 3-Trifluormethoxy-benzoylchlorid und 12 Gew.-% Ausgangsmaterial vor. Durch destillative Auftrennung wurden 37,5 g reines 3-Trifluormethoxy-benzoylchlorid mit einem Siedepunkt von 75 °C bei 13 mbar erhalten.

## Beispiel 4

In einer getrockneten Rührapparatur wurden 30 g 2-Difluormethoxy-trichlormethoxy-benzol vorgelegt und 1 ml Antimonpentachlorid versetzt. Es setzte eine leicht exotherme Reaktion ein. Anschließend wurde für 4 Stunden bei 80 °C gerührt. Gemäß gaschromatographischer Analyse lagen dann im Reaktionsgemisch 35 Gew.-% 2-Difluormethoxy-trifluormethoxy-benzol und 6 Gew.-% 2-Dichlormethoxytrifluormethoxy-benzol neben Verbindungen mit höherem Chlorgehalt vor. Durch Destillation wurden aus dem Reaktionsgemisch 7,2 g 2-Difluormethoxy-trifluormethoxy-benzol abgetrennt (Siedepunkt : 88 bis 89 °C bei 200 mbar).

## Beispiel 5

Zu 50 g 6-Nitro-2-chlor-2,3,3-trifluor-benzo-1,4-dioxen wurden 3 Tropfen Antimonpentachlorid gegeben und anschließend unter Feuchtigkeitsausschluß für 3 Stunden auf 140 °C erhitzt. Die gaschromatographische Analyse des Reaktionsgemisches zeigt dann einen Gehalt von 24,8 Gew.-% 6-Nitro-2,2,3,3-tetrafluor-benzo-1,4-dioxen neben 51,2 Gew.-% Ausgangsmaterial und 23,2 Gew.-% 6-Nitro-dichlor-difluor-benzo-1,4-dioxen an. Aus dem Reaktionsgemisch wurden 9,7 g 6-Nitro-2,2,3,3-Tetrafluor-benzo-1,4-dioxen mit einem Siedepunkt von 110 bis 112 °C bei 18 mbar und mit einem Brechungsindex von $n_D^{20}$ von 1,4750 abgetrennt.

## Beispiel 6

60 g 2-Methyl-5-nitrophenylthio-chlordifluormethan wurden mit 2 Tropfen Antimonpentachlorid für 5 Stunden auf 90 °C erhitzt. Danach wurden durch Destillation aus dem Reaktionsgemisch 23 g 2-Methyl-5-nitrophenylthiotrifluormethan mit einem Siedepunkt von 120 °C bei 20 mbar und mit einem Brechnungsindex von $n_D^{20}$ von 1,5205 abgetrennt. Im Destillationsrückstand befand sich neben wenig Ausgangsmaterial hauptsächlich der Dichlorfluormethylthioether.

## Beispiel 7

Zu einer Mischung aus 2 g Aluminiumchlorid und 1 g Natriumchlorid wurden 50 g 2,4-Dichlorphenylchlordifluormethylether zugegeben und für 2 Stunden auf 120 °C erhitzt. Eine gaschromatographische Analyse zeigte, daß das Reaktionsgemisch dann 34,4 % 2,4-Dichlorphenyl-trifluormethylether und 25,5 % unumgesetztes Ausgangsmaterial enthielt.

Beispiel 8

In einer Rührapparatur, die mit Innenthermometer und einer mit Wilsonspiralen gefüllten Kolonne mit Rückflußteiler versehen war, wurden 200 g 2-Chlor-2,3,3-trifluor-1,4-benzodioxen, 2,9 g Natriumchlorid und 6,6 g Aluminiumchlorid vorgelegt und auf 180 bis 205 °C Innentemperatur erhitzt. Dabei wurde am Kolonnenkopf eine 1. Fraktion in einer Menge von 108 g mit einem Siedepunkt von 145 bis 158 °C bei Normaldruck und einem Brechungsindex von $n_D^{20}$ von 1,4295 abgenommen. Danach wurde eine 2. Fraktion, im wesentlichen bestehend aus Ausgangsmaterial und anschließend, bei vermindertem Druck, eine 3. Fraktion bei 93 bis 94 °C und 20 mbar erhalten, die im wesentlichen aus 2,2-Dichlor-3,3-difluor-1,4-benzodioxen ($n_D^{20}$: 1,4990) bestand. Die 1. Fraktion wurde nochmals destilliert, wobei 76 g Tetrafluorbenzodioxen bei 144 bis 147 °C und 1 030 mbar mit einem Brechungsindex von 1,4220 erhalten wurden.

Beispiel 9 (zum Vergleich)

In einer Fluorierungsapparatur wurden 500 g Fluorwasserstoff, 5 ml Antimonpentachlorid und 150 g 2-Chlor-2,3,3-trifluor-1,4-benzodioxen für 6 Stunden auf 140 °C erhitzt. Danach wurde überschüssiger Fluorwasserstoff durch Destillation entfernt und der Rückstand einer fraktionierten Destillation unterworfen. Es wurden 27 g 2,2,3,3,-Tetrafluor-1,4-benzodioxen mit einem Siedepunkt von 142 bis 146 °C erhalten. Der Rückstand bestand aus einer verharzten Masse. Es konnte kein Ausgangsprodukt mehr isoliert werden.

**Patentansprüche**

1. Verfahren zur Herstellung von aromatischen Verbindungen, die über ein Heteroatom gebundene perfluorierte Seitenketten enthalten, dadurch gekennzeichnet, daß man aromatische Verbindungen, die über Sauerstoff-, Schwefel- und/oder Stickstoffatome gebundene perhalogenierte, aber nur partiell fluorierte Seitenketten enthalten, mit einem Lewis-Säure-Katalysator behandelt.

2. Verfahren nach Anspruch 1, dadurch gekennzeichnet, daß man ein Ausgangsmaterial einsetzt, das zusätzlich zu den über Sauerstoff-, Schwefel- und/oder Stickstoffatome gebundenen perhalogenierten, aber nur partiell fluorierten Seitenketten Alkyl-, Aryl-, Carbonsäurefluorid-, Carbonsäurechlorid-, Nitro-, Sulfochlorid-, Cyanid-, O-Alkyl-, O-Aryl- und/oder Halogen-Gruppen enthält und/oder mit einem carbocyclischen Ring substituiert ist.

3. Verfahren nach Ansprüchen 1 und 2, dadurch gekennzeichnet, daß man ein Ausgangsmaterial einsetzt, bei dem die über Sauerstoff-, Schwefel- und/oder Stickstoffatome gebundenen perhalogenierten, aber nur partiell fluorierten Seitenketten als Zyklus vorliegen.

4. Verfahren nach Ansprüchen 1 bis 3, dadurch gekennzeichnet, daß man als Katalysator Aluminiumtrichlorid, Aluminiumtribromid, Bortrichlorid, Bortrifluorid, Arsenpentachlorid, Antimonpentachlorid, Molybdänpentachlorid, Titantetrachlorid und/oder Eisentrichlorid einsetzt.

5. Verfahren nach Ansprüchen 1 bis 4, dadurch gekennzeichnet, daß man den Katalysator in Mengen von 0,001 bis 0,1 Mol, bezogen auf 1 Mol aromatische Verbindung einsetzt.

6. Verfahren nach Ansprüchen 1 bis 5, dadurch gekennzeichnet, daß man es bei Temperaturen im Bereich von 0 bis 150 °C durchführt.

7. Verfahren nach Ansprüchen 1 bis 6, dadurch gekennzeichnet, daß man unter Ausschluß von Feuchtigkeit arbeitet.

8. Verfahren nach Ansprüchen 1 bis 7, dadurch gekennzeichnet, daß man das erhaltene Reaktionsgemisch durch Destillation aufarbeitet.

**Claims**

1. Process for the preparation of aromatic compounds which contain perfluorinated side-chains bonded *via* a heteroatom, characterised in that aromatic compounds which contain side-chains which are perhalogenated, but only partially fluorinated, and bonded *via* oxygen, sulphur and/or nitrogen atoms, are treated with a Lewis acid catalyst.

2. Process according to Claim 1, characterised in that a starting material is used which contains, in addition to the side-chains which are perhalogenated, but only partially fluorinated, and bonded *via* oxygen, sulphur and/or nitrogen atoms, alkyl, aryl, carboxylic acid fluoride, carboxylic acid chloride, nitro, sulphochloride, cyanide, O-alkyl, O-aryl and/or halogen groups, and/or is substituted by a carbocyclic ring.

3. Process according to Claims 1 and 2, characterised in that a starting material is used in which the side-chains which are perhalogenated, but only partially fluorinated, and bonded *via* oxygen, sulphur and/or nitrogen atoms are present in the form of a cyclic structure.

4. Process according to Claims 1 to 3, characterised in that aluminium trichloride, aluminium

tribromide, boron trichloride, boron trifluoride, arsenic pentachloride, antimony pentachloride, molybdenum pentachloride, titanium tetrachloride and/or iron trichloride are used as the catalyst.

5. Process according to Claims 1 to 4, characterised in that the catalyst is used in quantities of 0.001 to 0.1 mol, relative to 1 mol of aromatic compound.

6. Process according to Claims 1 to 5, characterised in that it is carried out at temperatures in the range from 0 to 150 °C.

7. Process according to Claims 1 to 6, characterised in that it is carried out in the absence of moisture.

8. Process according to Claims 1 to 7, characterised in that the reaction mixture obtained is worked up by distillation.

**Revendications**

1. Procédé de production de composés aromatiques qui contiennent des chaînes latérales perfluorées liées par un hétéroatome, caractérisé en ce qu'on traite avec un catalyseur à base d'un acide de Lewis des composés aromatiques qui contiennent des chaînes latérales perhalogénées, mais partiellement fluorées seulement, liées par des atomes d'oxygène, de soufre et/ou d'azote.

2. Procédé suivant la revendication 1, caractérisé en ce qu'on utilise une matière de départ qui contient, en plus des chaînes latérales perhalogénées mais partiellement fluorées seulement, liées par des atomes d'oxygène, de soufre et/ou d'azote, des groupes alkyle, aryle, fluorure d'acide carboxylique, chlorure d'acide carboxylique, nitro, sulfochlorure, cyanure, O-alkyle, O-aryle et/ou halogéno et/ou qui est substituée par un noyau carbocyclique.

3. Procédé suivant les revendications 1 et 2, caractérisé en ce qu'on utilise une matière de départ dans laquelle les chaînes latérales perhalogénées mais partiellement fluorées seulement, liées par des atomes d'oxygène, de soufre et/ou d'azote, se présentent comme cycle.

4. Procédé suivant les revendications 1 à 3, caractérisé en ce qu'on utilise comme catalyseur le trichlorure d'aluminium, le tribromure d'aluminium, le trichlorure de bore, le trifluorure de bore, le pentachlorure d'arsenic, le pentachlorure d'antimoine, le pentachlorure de molybdène, le tétrachlorure de titane et/ou le trichlorure de fer.

5. Procédé suivant les revendications 1 à 4, caractérisé en ce qu'on utilise le catalyseur en quantités de 0,001 à 0,1 mole, par rapport à une mole de composé aromatique.

6. Procédé suivant les revendications 1 à 5, caractérisé en ce qu'il est mis en œuvre à des températures comprises dans l'intervalle de 0 à 150 °C.

7. Procédé suivant les revendications 1 à 6, caractérisé en ce qu'on opère à l'abri de l'humidité.

8. Procédé suivant les revendications 1 à 7, caractérisé en ce que le mélange réactionnel obtenu est traité par distillation.